# EUROPEAN PATENT APPLICATION

(11) **EP 4 740 855 A1**
(43) Date of publication of application: **13.05.2026**
(21) Application number: 24855766.2
(22) Date of filing: 19.08.2024
(51) Int. Cl.: A61B 5/145, A61B 5/00

(54) **APPLICATOR, STERILIZATION UNIT, AND ANALYTE SENSOR**

(30) Priority: 23.08.2023 CN 202311073067; 13.10.2023 CN 202311331942
(71) Applicant: Shanghai United Imaging Microelectronics Technology Co., Ltd., Shanghai 201899 (CN)
(72) Inventor: LIU, Wei, Shanghai 201899 (CN)
(74) Representative: Murgitroyd & Company
(86) International application number: PCT/CN2024/113000
(87) International publication number: WO 2025/040043

(57) **Abstract**

An applicator (100), comprising: a first housing (110); a second housing (120) partially accommodated in the first housing (110); and a pushing block (130) slidably connected to the second housing (120). A trigger member (111) is provided on the side of the top wall (1101) of the first housing (110) facing the second housing (120), a limiting member (121) is provided on the second housing (120), and a trigger matching member (131) is provided on the pushing block (130). The trigger matching member (131) can abut against both the trigger member (111) and the limiting member (121), and under the driving of the first housing (110), the trigger member (111) can drive the trigger matching member (131) to be separated from the limiting member (121). Also disclosed are a sterilization unit and an applicator (100) comprising the sterilization unit.

## Description

### CROSS-REFERENCE TO RELATED APPLICATIONS

This application claims the priority benefit of Chinese patent application No. 202311073067.3 filed with the China National Intellectual Property Administration on August 23, 2023, and Chinese patent application No. 202311331942.3 filed with the China National Intellectual Property Administration on October 13, 2023. The entire disclosures of the above identified Chinese patent applications are incorporated by reference herein for all purposes.

### TECHNICAL FIELD

The present disclosure relates to the field of medical device technologies, and in particular, to an applicator and a sterilization unit.

### BACKGROUND

An applicator is a tool used to assist in attaching an analyte sensor to the skin of a user. An analyte sensor is a wearable device that can monitor changes in the concentration of bioanalytical substances in the interstitial fluid of subcutaneous tissue. When the analyte sensor is attached to the skin of the user, it can continuously obtain information related to the concentration of the analytical substance through a probe implanted in the subcutaneous tissue.

In conventional applicators, a button used to trigger the ejection and insertion of the analyte sensor is usually exposed on the applicator. Since the setting of the button provides the user with an obvious guiding direction to touch and press the button, additional safety devices need to be provided for the button to prevent the improper ejection and insertion of the analyte sensor caused by the user's accidental contact. This will increase the additional operation of unlocking the safety device. In addition, the button exposed on the applicator will cause the overall appearance of the applicator to be discontinuous and abrupt, thereby affecting the overall external aesthetics of the applicator.

The part of the probe of the analyte sensor that is to be implanted into the subcutaneous tissue needs to maintain sterility before being implanted into the subcutaneous tissue. Conventional analyte sensors are usually provided with a bacteria barrier to isolate this part from the external environment before use, and during use, the user needs to remove the bacteria barrier to expose the implantable part.

Some conventional analyte sensors adopt irradiation sterilization. During the sterilization process, the probe placed in the sealed area is irradiated by electron beams passing through the sealed cover. The sealed area is usually sealed by the pressing force between the sealed cover of the applicator and the housing, i.e., the seal can only be guaranteed when the sealed cover is secured to the housing. Therefore, during irradiation sterilization, the entire applicator equipped with the analyte sensor needs to be placed into the sterilization equipment for sterilization. The applicator occupies a large space, resulting in low sterilization efficiency.

### SUMMARY

In a first aspect of the present disclosure, an applicator is provided, which includes a first housing including a top wall, a second housing partially accommodated in the first housing, and a pushing block slidably connected to the second housing. A trigger member is provided on a side of the top wall of the first housing facing the second housing, and a limiting member is provided on the second housing. A trigger mating member is provided on the pushing block. The trigger mating member is capable of abutting against both the trigger member and the limiting member, and the trigger member is capable of driving the trigger mating member to be disengaged from the limiting member when driven by the first housing.

In the above first aspect, the trigger member is in positional interference with the trigger mating member. The trigger mating member has a first state and a second state. In the first state, the trigger mating member is limited by the limiting member. In the second state, the trigger mating member is disengaged from the limiting member, and preferably, the trigger mating member is disengaged from the limiting member when pushed by the trigger member.

In the above first aspect, one of the trigger member and the trigger mating member is configured with a hook, and the other of the trigger member and the trigger mating member is configured with a groove. The hook is capable of being engaged with the groove. The hook is protrudingly provided on one of the trigger member and the trigger mating member, and the groove is recessedly provided on the other of the trigger member and the trigger mating member.

In the above first aspect, a fastening member is further provided on the top wall, and a fastening mating member is provided on the second housing. The fastening member abuts against the fastening mating member in the movement direction of the trigger member, and the fastening member is capable of driving the fastening member and/or the fastening mating member to elastically deform when driven by the top wall.

In the above first aspect, a first positioning member is further provided in the first housing, and a first positioning mating member is provided on the second housing. The first positioning member is in sliding fit with the first positioning mating member in the movement direction of the trigger member.

In the above first aspect, a second positioning member is further provided on the pushing block, and a second positioning mating member is provided on the second housing. The second positioning member is in sliding fit with the second positioning mating member in the movement direction of the trigger member.

In the above first aspect, the applicator further includes a retraction block movably mounted on the pushing block. The analyte sensor has a probe, the retraction block is provided with an auxiliary needle, the auxiliary needle is formed with an accommodating cavity, and the probe is at least partially accommodated in the accommodating cavity.

In the above first aspect, the applicator further includes a second elastic member mounted between the retraction block and the pushing block in a pre-compressed manner. An elastic claw is further provided on the pushing block, and the elastic claw is capable of abutting against the inner peripheral wall of the second housing. The elastic claw is in positional interference with the retraction block, and the elastic claw is configured to elastically deform in a direction away from the retraction block in response to pushing by the retraction block.

In the above first aspect, the elastic claw has a pushing inclined surface. The pushing inclined surface abuts against the retraction block in the movement direction of the retraction block relative to the pushing block. A sliding groove is provided on the outer peripheral wall of the retraction block. The elastic claw is in sliding fit with the sliding groove in the movement direction of the retraction block relative to the pushing block.

In a second aspect of the present disclosure, a sterilization unit is provided, which includes an auxiliary needle, an analyte sensor, and an accommodating member. The auxiliary needle includes a handle portion and a sharp portion. The handle portion includes a handle section and a connecting section, and the tip of the sharp portion is away from the connecting section. The analyte sensor includes an application portion and a probe secured to each other, and the application portion is provided with a through hole. The handle section forms a seal with the through hole on a side close to the handle section, and the connecting section at least partially extends through the through hole. The accommodating member is located on a side of the analyte sensor away from the handle section, and the accommodating member is provided with an accommodating space capable of accommodating the sharp portion and the probe. The accommodating member is removably secured to the connecting section, and the accommodating member forms a seal with the through hole on a side away from the handle section.

In the above second aspect, the sterilization unit further includes a base member located on a side of the application portion away from the handle portion, and the accommodating member is at least partially inserted through the base member.

In the above second aspect, the accommodating member is integrally formed with the base member or secured to the base member.

In the above second aspect, one of the accommodating member and the base member is provided with a first guide member, and the other of the accommodating member and the base member is provided with a second guide member cooperating with the first guide member. When the accommodating member is secured to the connecting section, the first guide member abuts against the second guide member.

In the above second aspect, an inner wall of the accommodating space is provided with a first engagement portion at an end close to the analyte sensor, and the connecting section can be inserted into the accommodating space and engaged with the first engagement portion.

In the above second aspect, the accommodating space extends through the accommodating member, and the accommodating member further includes a sealing portion capable of being secured to the accommodating member to seal an opening of the accommodating space at an end away from the analyte sensor.

In the above second aspect, a shielding cavity capable of mounting a shielding block is provided on a side of the base member away from the analyte sensor.

In the above second aspect, the base member has a shielding layer, and a projection of the shielding layer in the axial direction of the accommodating member completely covers the analyte sensor.

In a third aspect of the present disclosure, an applicator is provided, which includes a housing, a bottom cover removably secured to the housing, and the sterilization unit according to the above second aspect accommodated inside the housing and the bottom cover. The handle section and the analyte sensor are respectively removably secured to the housing, and the accommodating member is relatively fixed to the bottom cover.

In the above third aspect, when the sterilization unit includes a base member, the base member is capable of limiting the movement of the accommodating member toward the side close to the analyte sensor along its own axial direction. An inner wall of the bottom cover is provided with a second engagement portion, and an outer periphery of the base member is provided with a third engagement portion. When the housing is secured to the bottom cover, the second engagement portion is engaged with the third engagement portion.

In a fourth aspect of the present disclosure, an applicator is provided, which includes a first housing including a top wall, a second housing partially accommodated in the first housing, a pushing block slidably connected to the second housing, and the sterilization unit according to the above second aspect mounted on the pushing block. A trigger member is provided on a side of the top wall of the first housing facing the second housing, and a limiting member is provided on the second housing. A trigger mating member is provided on the pushing block. The trigger mating member is capable of abutting against both the trigger member and the limiting member, and the trigger member is capable of driving the trigger mating member to be disengaged from the limiting member when driven by the first housing.

In a fifth aspect of the present disclosure, an applicator with a sterilization unit is provided, which includes the applicator according to the above first aspect and the sterilization unit according to the above second aspect.

### BRIEF DESCRIPTION OF THE DRAWINGS

To more clearly illustrate the technical solutions in the embodiments of the present disclosure or the conventional technology, the following will briefly introduce the drawings needed in the description of the embodiments or the conventional technology. Apparently, the drawings in the following description are only some embodiments of the present disclosure. For those of ordinary skill in the art, other drawings can be obtained according to these drawings without creative efforts.
FIG. 1 is a schematic diagram of a structure of an applicator according to an embodiment of the present disclosure.
FIG. 2 is an exploded view of the applicator shown in FIG. 1.
FIG. 3 is a schematic diagram of a structure of the first housing in FIG. 2.
FIG. 4 is a schematic diagram of a structure of an assembly formed by assembling the second housing and the pushing block shown in FIG. 2.
FIG. 5 is a schematic diagram of a structure of an assembly formed by assembling the pushing block, the analyte sensor, and the retraction block shown in FIG. 2.
FIG. 6 is a schematic diagram of a structure of an assembly formed by assembling the pushing block and the analyte sensor shown in FIG. 2.
FIG. 7 is a schematic diagram of a structure of the retraction block shown in FIG. 2.
FIG. 8 is a cross-sectional diagram of a structure of the applicator according to an embodiment of the present disclosure in an initial state.
FIG. 9 is an enlarged view of part P shown in FIG. 8.
FIG. 10 is a cross-sectional diagram of a structure of the applicator according to an embodiment of the present disclosure when the top wall of the first housing is pressed.
FIG. 11 is a cross-sectional diagram of a structure of the applicator according to an embodiment of the present disclosure when the pushing block is completely ejected from the second housing.
FIG. 12 is an enlarged view of part Q shown in FIG. 11.
FIG. 13 is a cross-sectional diagram of a structure of the applicator according to an embodiment of the present disclosure when the retraction block completes retraction.
FIG. 14 is a schematic diagram showing the applicator applied to a user according to an embodiment of the present disclosure.
FIG. 15 is a schematic diagram showing the analyte sensor attached to a user according to an embodiment of the present disclosure.
FIG. 16 is an exploded view of an applicator with a sterilization unit according to an embodiment of the present disclosure.
FIG. 17 is an exploded view of the sterilization unit according to an embodiment of the present disclosure.
FIG. 18 is a cross-sectional diagram of a structure of the sterilization unit shown in FIG. 16.
FIG. 19 is a schematic diagram of a structure of the accommodating member shown in FIG. 17.
FIG. 20 is a cross-sectional diagram of a structure of the accommodating member shown in FIG. 18.
FIG. 21 is a schematic diagram of a structure of the base member shown in FIG. 17 from a bottom view.
FIG. 22 is a schematic diagram of a structure of the bottom cover shown in FIG. 16 from another angle.
FIG. 23 is a cross-sectional diagram of a structure of an assembled applicator with a sterilization unit according to an embodiment of the present disclosure.
FIG. 24 is a cross-sectional diagram of a structure of the applicator shown in FIG. 23 with the housing and the bottom cover being separated.

### DETAILED DESCRIPTION OF THE EMBODIMENTS

In order to make the above objectives, technical solutions, and advantages of the present disclosure clearer, the technical solutions in the present disclosure will be clearly and completely described below with reference to the accompanying drawings in the present disclosure. Apparently, the described embodiments are some embodiments of the present disclosure, but not all embodiments. Based on the embodiments in the present disclosure, all other embodiments obtained by those of ordinary skill in the art without creative efforts shall fall within the protection scope of the present disclosure.

When a feature or element is referred to as being "on" another feature or element, it can be directly on the other feature or element, or there may be intermediate features and/or elements. In comparison, when a feature or element is referred to as being "directly on" another feature or element, there are no intermediate features or elements. It will also be understood that when a feature or element is referred to as being "connected", "attached" or "coupled" to another feature or element, it can be directly connected, attached or coupled to the other feature or element, or there may be intermediate features or elements. In comparison, when a feature or element is referred to as being "directly connected", "directly attached" or "directly coupled" to another feature or element, there are no intermediate features or elements.

For ease of description, spatial relative terms such as "below", "under", "above", "over" may be used herein to describe the relationship between one feature or element and another feature or element as shown in the figures. It will be understood that the spatial relative terms are intended to encompass different orientations of the device in use or operation in addition to the orientation depicted in the figures. For example, if the device in the figures is inverted, features or elements described as "below" other features or elements will then be oriented "above" the other features or elements. Thus, the exemplary term "below" can encompass both an orientation of above and below. It will also be understood that the device may be oriented in other ways (e.g., rotated 90 degrees or at other angles) and that the spatially relative descriptions used herein will be interpreted accordingly.

Although the terms "first" and "second" may be used herein to describe various features or elements, these features or elements should not be limited by these terms unless otherwise specified. These terms may be used to distinguish one feature or element from another feature or element. Thus, a first feature or element described below may be referred to as a second feature or element, and similarly, a second feature or element described below may be referred to as a first feature or element without departing from the scope of the present disclosure.

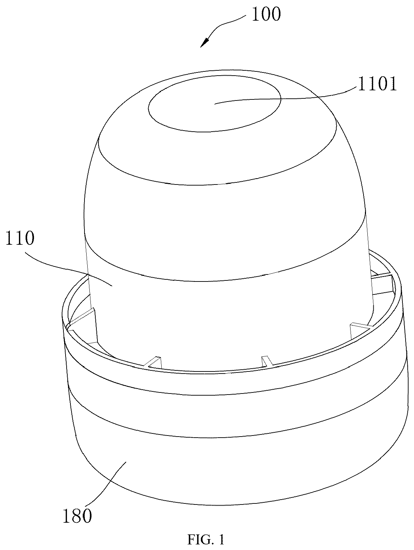

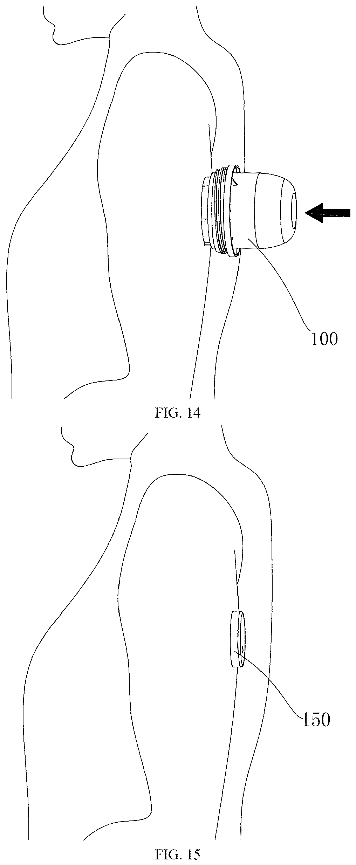

As shown in FIG. 1, in a first aspect of the present disclosure, an applicator 100 for assisting in attaching an analyte sensor to a user's body is provided. Referring to FIGS. 14 and 15, to attach the analyte sensor 150 with a probe 151 to a target position on the user's body, such as the user's arm, the applicator 100 equipped with the analyte sensor 150 is first applied to the target position. Then, the top wall 1101 of the first housing 110 of the applicator 100 is pressed in a direction indicated by an arrow in FIG. 14, and the analyte sensor 150 is released from the applicator 100 to contact the user's skin, so that the probe 151 of the analyte sensor 150 is implanted into the user's subcutaneous tissue.

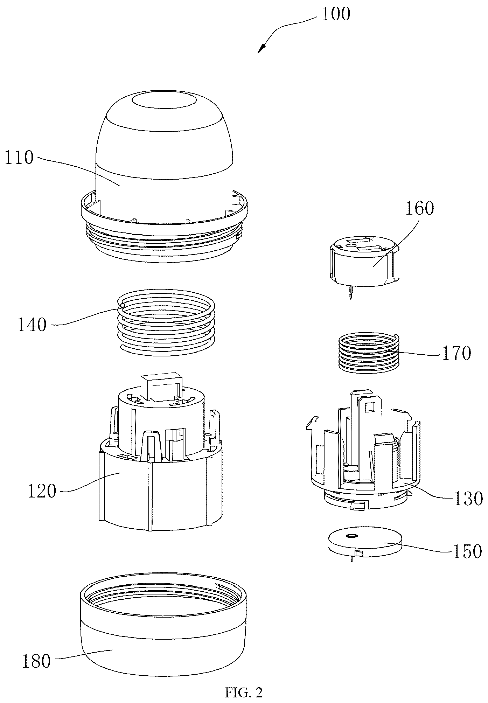

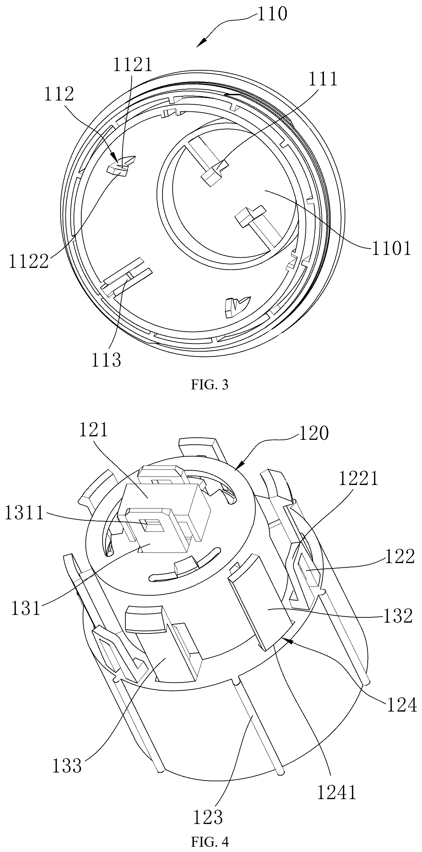

Referring to FIG. 2, the applicator 100 includes a first housing 110, a second housing 120, a pushing block 130, a first elastic member 140, an analyte sensor 150, a retraction block 160, a second elastic member 170, and a housing cover 180. Referring to FIGS. 3, 8, and 9, the first housing 110 includes a top wall 1101, the second housing 120 is partially accommodated in the first housing 110, a trigger member 111 is provided on a side of the top wall 1101 of the first housing 110 facing the second housing 120, and a limiting member 121 is provided on the second housing 120. The pushing block 130 is slidably connected to the second housing 120. A trigger mating member 131 is provided on the pushing block 130, and the first elastic member 140 is mounted between the pushing block 130 and the first housing 110 in a pre-compressed manner. The analyte sensor 150 is mounted on the pushing block 130. The trigger mating member 131 is capable of abutting against both the trigger member 111 and the limiting member 121 to lock the pushing block 130 to the second housing 120. The trigger member 111 is capable of driving the trigger mating member 131 to be disengaged from the limiting member 121 when driven by the first housing 110, so that the first elastic member 140 in its pre-compressed state can push the pushing block 130, thereby driving the analyte sensor 150 to eject from the second housing 120.

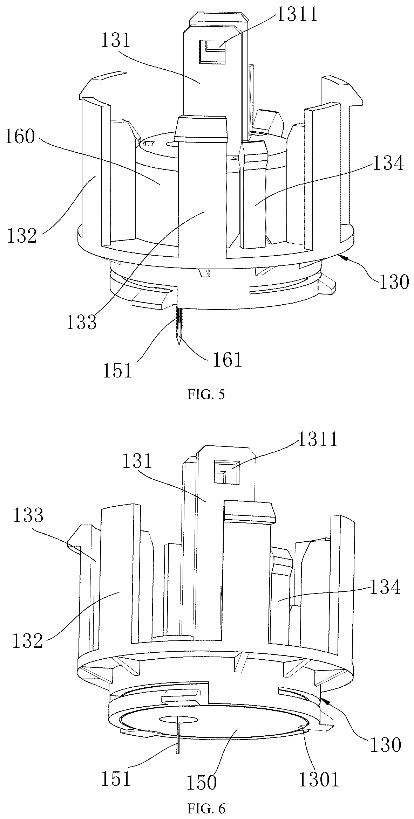

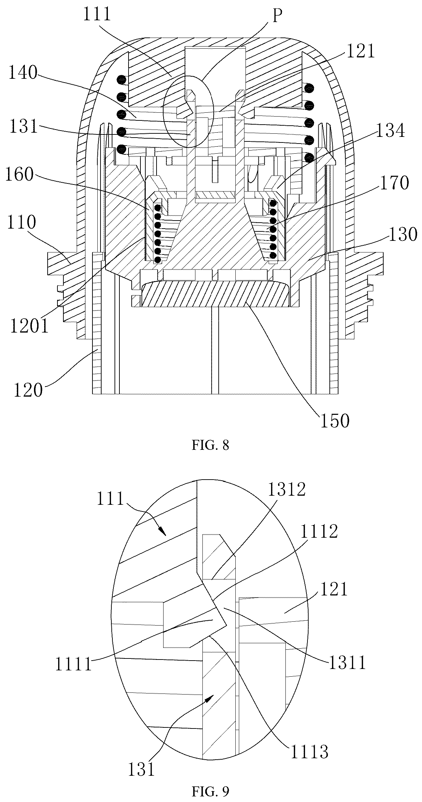

Referring to FIG. 6, the analyte sensor 150 can be embedded in the pushing block 130 and limited by a convex buckle 1301 on the pushing block 130. In this way, the analyte sensor 150 can be prevented from falling off the pushing block 130 under the action of gravity. Continuing to refer to FIG. 8, when the pushing block 130 is locked on the second housing 120, the first elastic member 140 remains in a pre-compressed state between the first housing 110 and the pushing block 130. On a condition that the trigger mating member 131 is disengaged from the limiting member 121 when pushed by the trigger member 111 and the abutment between the limiting member 121 and the trigger mating member 131 is released, the locking of the pushing block 130 on the second housing 120 can be released. In this case, the pre-compressed first elastic member 140 can push the pushing block 130, so that the pushing block 130 drives the analyte sensor 150 to eject from the second housing 120.

It can be understood that by arranging the trigger member 111 for controlling the locking/unlocking between the pushing block 130 and the second housing 120 on the side of the top wall 1101 of the first housing 110 facing the second housing 120, and using the pre-compressed first elastic member 140 to drive the pushing block 130 and the analyte sensor 150 to eject, no button is provided on the exterior of the applicator 100. This not only eliminates the need to set an unlocking safety device for the matching button but also maintains the continuity and aesthetics of the appearance of the applicator 100. In addition, an unlocking control between the second housing 120 and the pushing block 130 is independent of the ejection of the pushing block 130 driving the analyte sensor 150 from the second housing 120, which can improve the stability of the user when using the applicator 100, and will not affect the ejection of the pushing block 130 from the second housing 120 due to different pressing forces when pressing the top wall 1101 of the first housing 110.

Referring to FIGS. 8 and 9, the trigger member 111 is in positional interference with the trigger mating member 131. The trigger mating member 131 has a first state and a second state. In the first state, the trigger mating member 131 is limited by the limiting member 121. In this case, the pushing block 130 is capable of being locked to the second housing 120 through the cooperation between the trigger mating member 131 and the trigger member 111. In the second state, the trigger mating member 131 is capable of being disengaged from the limiting member 121 when pushed by the trigger member 111. In this case, the trigger mating member 131 is capable of elastically deforming relative to the trigger member 111 when pushed by the trigger member 111 to be disengaged from the trigger member 111 and release the locking of the pushing block 130 on the second housing 120. Specifically, the locking between the pushing block 130 and the second housing 120 is achieved through the abutment between the trigger mating member 131 and the limiting member 121. On a condition that the trigger mating member 131 moves relative to the limiting member 121 when pushed by the trigger member 111, the trigger mating member 131 is capable of being disengaged from the limiting member 121, to achieve the purpose of releasing the locking of the pushing block 130 on the second housing 120.

It should be noted that the above-described limiting member 121 is capable of abutting against the trigger mating member 131, and specifically, the part of the trigger mating member 131 passing through the second housing 120 is capable of abutting against the limiting member 121. In the embodiment, the number of trigger mating members 131 is two, and two trigger mating members 131 are symmetrically arranged on both sides of the limiting member 121.

One of the trigger member 111 and the trigger mating member 131 may be configured with a hook, and the other of the trigger member 111 and the trigger mating member 131 may be configured with a groove. The hook is capable of being engaged with the groove to form positional interference.

Exemplarily, referring to FIG. 9, in the embodiment, a hook 1111 is protrudingly provided on the trigger member 111, and a groove 1311 is recessedly provided on the trigger mating member 131. A first inclined surface 1112 and a second inclined surface 1113 are provided on the hook 1111. The first inclined surface 1112 and the second inclined surface 1113 are arranged on both sides of the hook 1111 and can respectively abut against the groove wall 1312 of the groove 1311. In this way, the pushing block 130 has a tendency to move relative to the first housing 110 when pushed by the pre-compressed first elastic member 140, so that the first inclined surface 1112 of the hook 1111 abuts against the corresponding groove wall 1312 of the groove 1311 on the trigger mating member 131. This abutment drives the trigger mating member 131 to have a tendency to elastically deform toward the limiting member 121. In this case, the limiting member 121 abuts against the trigger mating member 131 and limits the elastic deformation of the trigger mating member 131, thereby locking the pushing block 130 on the first housing 110. Meanwhile, the locking of the pushing block 130 on the first housing 110 is achieved by the abutment between the first inclined surface 1112 on the hook 1111 and the corresponding groove wall 1312 on the groove 1311, and the first elastic member 140 is kept in a pre-compressed state. Then, when the user presses the top wall 1101 of the first housing 110 and drives the trigger member 111 to move, the second inclined surface 1113 of the hook 1111 on the trigger member 111 can push the corresponding groove wall 1312 of the groove 1311 on the trigger mating member 131, so that the pushing block 130 moves relative to the second housing 120 in the movement direction of the trigger member 111, and the trigger mating member 131 is disengaged from the limiting member 121. During this process, the trigger mating member 131 will elastically deform toward the limiting member 121 and be disengaged from the hook 1111, thereby releasing the locking between the pushing block 130 and the first housing 110. In this way, the pre-compressed first elastic member 140 can push the pushing block 130, thereby driving the analyte sensor 150 to eject from the second housing 120. Alternatively, in other embodiments, the hook may be protrudingly provided on the trigger mating member 131, and the groove may be recessedly provided on the trigger member 111.

It should be noted that when the user uses the applicator 100, the applicator 100 needs to first abut against the user's skin, i.e., the second housing 120 is attached to and limited by the user's skin. When the pushing block 130 is ejected downward from the second housing 120 when pushed by the pre-compressed first elastic member 140, the pushing block 130 moves toward the user's skin and is disengaged from the second housing 120.

Referring to FIGS. 3 and 4, a fastening member 112 is further provided on the top wall 1101, and a fastening mating member 122 is provided on the second housing 120. The fastening member 112 abuts against the fastening mating member 122 in the movement direction of the trigger member 111, and the fastening member 112 is capable of driving the fastening member 112 and/or the fastening mating member 122 to elastically deform when driven by the top wall 1101. Specifically, when the top wall 1101 drives the trigger member 111 to move, it is necessary to overcome the abutment limit between the fastening member 112 and the fastening mating member 122. That is, the pressing force of the user when pressing the top wall 1101 needs to be greater than the force required for the elastic deformation of the above-described fastening member 112 and/or the fastening mating member 122. In this way, the applicator 100 can be designed differently according to different user needs, making it easier for the user to operate.

Exemplarily, the fastening member 112 is capable of driving the fastening mating member 122 to elastically deform when driven by the top wall 1101. As a result, the structural strength of the fastening mating member 122 can be adjusted by changing the size, wall thickness, etc. of the fastening mating member 122, thereby enabling the pushing block 130 to be ejected from the second housing 120 under different pressing forces of the applicator 100. Those skilled in the art can understand that in other embodiments, when driven by the top wall 1101, the fastening member 112 can also elastically deform, or both the fastening member 112 and the fastening mating member 122 can elastically deform at the same time.

Continuing to refer to FIGS. 3 and 4, the fastening mating member 122 is configured as a doorframe structure, and the fastening member 112 has a plurality of convex teeth 1121 arranged in sequence in the movement direction of the trigger member 111. When the applicator 100 is in the initial state, the beam 1221 of the fastening mating member 122 is clamped between two adjacent convex teeth 1121, so that the assembly positioning of the first housing 110 on the second housing 120 can be realized, and the first housing 110 can be prevented from being separated from the second housing 120. The fastening member 112 is capable of pushing the fastening mating member 122 with the inclined surface 1122 of the convex tooth 1121 when driven by the top wall 1101. By pushing the inclined surface 1122 against the fastening mating member 122, the fastening mating member 122 is caused to elastically deform, thereby enabling the convex tooth 1121 of the fastening member 112 located outside the beam 1221 to bypass the beam 1221 and move into the fastening mating member 122. Those skilled in the art can understand that the fastening member 112 can also be configured as a doorframe structure, and the convex teeth are arranged on the fastening mating member 122 in sequence in the movement direction of the trigger member 111.

As can be seen from the above, the fastening member 112 corresponds to the fastening mating member 122 one by one. The number of fastening members 112 on the first housing 110 of the present disclosure is specifically set to three, and the three fastening members 112 are arranged at intervals in the circumferential direction of the first housing 110. Those skilled in the art can understand that in other embodiments, the number of fastening members 112 can also be set to two, four, five, or even more.

Continuing to refer to FIGS. 3 and 4, a first positioning member 113 is further provided in the first housing 110, and a first positioning mating member 123 is provided on the second housing 120. The first positioning member 113 is in sliding fit with the first positioning mating member 123 in the movement direction of the trigger member 111. In this way, the relative rotation between the first housing 110 and the second housing 120 can be limited, so that when the user uses the applicator 100, the applicator 100 can only be triggered by pressing the top wall 1101 of the first housing 110.

Referring to FIGS. 4 to 6, a second positioning member 132 is further provided on the pushing block 130, and a second positioning mating member 124 is provided on the second housing 120. The second positioning member 132 is in sliding fit with the second positioning mating member 124 in the movement direction of the trigger member 111. In this way, the rotational movement of the pushing block 130 relative to the second housing 120 when the pushing block 130 is ejected from the second housing 120 can be limited.

Exemplarily, the second positioning member 132 is arranged to extend through the second housing 120, and the second positioning mating member 124 is configured as a through hole 1241 matching the second positioning member 132. The abutment between the second positioning member 132 and the hole wall (not shown) of the through hole 1241 provides the circumferential limit of the pushing block 130 on the second housing 120.

Referring to FIGS. 4 to 6, a limiting hook 133 is further provided on the pushing block 130. The limiting hook 133 is adapted to extend through the second housing 120, and the limiting hook 133 is capable of moving relative to the second housing 120 when driven by the pushing block 130 and hooking the second housing 120, thus limiting the maximum movement stroke of the pushing block 130 when the pushing block 130 is ejected from the second housing 120, so as to prevent the pushing block 130 from being separated from the second housing 120. By this means, during the process of the pushing block 130 driving the analyte sensor 150 to eject from the second housing 120, when the pushing block 130 can be limited to the second housing 120 through the limiting hook 133, the analyte sensor 150 will continue to move and be disengaged from the pushing block 130. In this way, the probe 151 of the analyte sensor 150 can be implanted into the user's subcutaneous tissue.

As can be seen from the above, the first elastic member 140 is applied in the applicator 100 to elastically push the pushing block 130. Therefore, the above-described first elastic member 140 can be set as a spring. Of course, for those skilled in the art, the first elastic member 140 can also be set as a rubber sleeve, a bellows, etc. with high elasticity.

It can be understood that the probe 151 of the analyte sensor 150 is used to be implanted into the user's subcutaneous tissue to detect the analyte in the user's body. The analyte includes one or more of glucose concentration, acetylcholine, amylase, bilirubin, cholesterol, human chorionic gonadotropin, creatine kinase (e.g., CK-MB), creatine, DNA, fructosamine, glucose, glutamine, growth hormone, hormones, ketones, lactate, peroxides, prostate-specific antigen, prothrombin, RNA, thyroid-stimulating hormone, or troponin. The analyte sensor 150 can also be configured to monitor the concentration of drugs, such as antibiotics (e.g., gentamicin, vancomycin, etc.), digitoxin, digoxin, abused drugs, theophylline, warfarin, and other drugs.

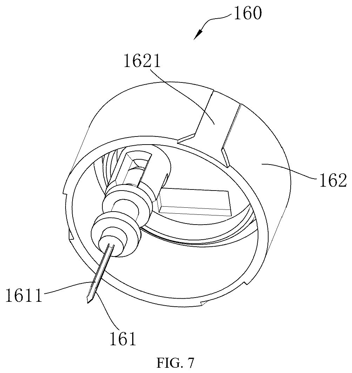

Referring to FIGS. 5 and 7, the applicator 100 further includes a retraction block 160 movably mounted on the pushing block 130. The retraction block 160 is provided with an auxiliary needle 161, the auxiliary needle 161 is formed with an accommodating cavity 1611, and the probe 151 of the analyte sensor 150 is at least partially accommodated in the accommodating cavity 1611. In this way, the probe 151 of the analyte sensor 150 can be protected during the implantation into the user's subcutaneous tissue, and the probe 151 is prevented from being damaged by pressure. It should be noted that the auxiliary needle 161 of the retraction block 160 extends outward relative to the probe 151. In this way, during the implantation of the probe 151 into the user's subcutaneous tissue, the auxiliary needle 161 can be inserted into the user's subcutaneous tissue first, which can effectively protect the probe 151.

Exemplarily, the retraction block 160 is mounted on a side of the pushing block 130 away from the analyte sensor 150, and the auxiliary needle 161 on the retraction block 160 is capable of passing through the pushing block 130 and at least partially wrap the probe 151, so that the pushing block 130 is capable of driving both the analyte sensor 150 and the retraction block 160 to eject from the second housing 120 when pushed by the first elastic member 140.

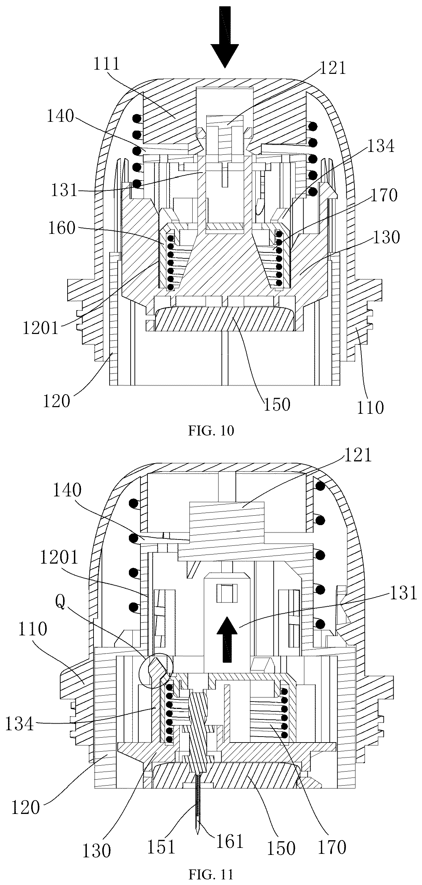

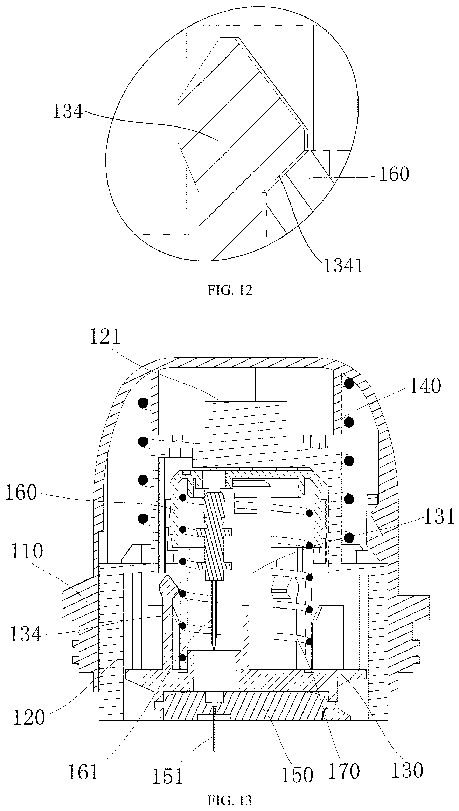

Referring to FIGS. 2, 8, 10, 11, and 13, the applicator 100 further includes a second elastic member 170 mounted between the retraction block 160 and the pushing block 130 in a pre-compressed manner. An elastic claw 134 is further provided on the pushing block 130, and the elastic claw 134 is capable of abutting against the inner peripheral wall 1201 of the second housing 120. The elastic claw 134 is in positional interference with the retraction block 160, and the elastic claw 134 is configured to elastically deform in a direction away from the retraction block 160 in response to pushing by the retraction block 160. In this way, the pre-compressed second elastic member 170 can be used to enable the automatic retraction of the retraction block 160 into the applicator 100, which can prevent physical harm and visual impact to the user due to the exposure of the auxiliary needle 161. Similar to the first elastic member 140, the second elastic member 170 includes but is not limited to a spring, a rubber sleeve with high elasticity, a bellows, etc.

Exemplarily, the elastic claw 134 has a pushing inclined surface 1341. The pushing inclined surface 1341 abuts against the retraction block 160 and forms positional interference in the movement direction of the retraction block 160 relative to the pushing block 130.

It can be understood that the retraction block 160 has a tendency to move relative to the pushing block 130 when pushed by the pre-compressed second elastic member 170, so that the elastic claw 134 has a tendency to elastically deform outward when driven by the retraction block 160. When the pushing block 130 compresses the first elastic member 140 and is accommodated in the second housing 120, the inner peripheral wall 1201 of the second housing 120 will abut against the elastic claw 134 and restrain the outward expansion and deformation of the elastic claw 134. In this way, when the applicator 100 is in the initial state, the elastic claw 134 of the pushing block 130 is limited to the retraction block 160 due to the abutment of the inner peripheral wall 1201 of the second housing 120. At the same time, the retraction block 160 will maintain the state of compressing the second elastic member 170 due to the limit of the pushing inclined surface 1341 on the elastic claw 134. When the pushing block 130 is ejected from the second housing 120 when pushed by the first elastic member 140, the elastic claw 134 will be disengaged from the inner peripheral wall 1201 of the second housing 120 when driven by the pushing block 130, and the lock of the inner peripheral wall 1201 on the outward expansion and deformation of the elastic claw 134 will be released. Then, the retraction block 160 can be separated from the pushing block 130 when pushed by the pre-compressed second elastic member 170, and the automatic retraction of the retraction block 160 can be achieved.

Referring to FIG. 7, a sliding groove 1621 is provided on the outer peripheral wall 162 of the retraction block 160. The elastic claw 134 is in sliding fit with the sliding groove 1621 in the movement direction of the retraction block 160 relative to the pushing block 130, so that the circumferential limit between the retraction block 160 and the pushing block 130 can be achieved.

Referring to FIG. 1, the applicator 100 further includes a housing cover 180 removably connected to the first housing 110. For example, the housing cover 180 can be assembled to the first housing 110 by means of threads or buckles. In this way, the first housing 110 can be covered with the housing cover 180, so that the entire applicator 100 can present a closed structure, which is convenient for the overall packaging and transportation of the applicator 100, and can also prevent the external environment from polluting the auxiliary needle 161 and the probe 151.

Referring to FIGS. 8, 10, 11, and 13, when the user uses the applicator 100, the trigger member 111 can be driven to move by pressing the top wall 1101 of the first housing 110. The pushing block 130 can be unlocked from the second housing 120 by pushing the trigger mating member 131 with the trigger member 111. In this way, the pushing block 130 is capable of driving the analyte sensor 150 and the retraction block 160 to eject from the second housing 120 under the pushing of the pre-compressed first elastic member 140, and the auxiliary needle 161 of the retraction block 160 and the probe 151 of the analyte sensor 150 can be implanted into the user's subcutaneous tissue. At the same time, the elastic claw 134 follows the pushing block 130 to be disengaged from the second housing 120, and the limit of the inner peripheral wall 1201 on the second housing 120 to the outward expansion and deformation of the elastic claw 134 is released, i.e., the locking between the retraction block 160 and the pushing block 130 is released. In this way, the retraction block 160 can be separated upward from the pushing block 130 under the pushing of the pre-compressed second elastic member 170 and retracted into the second housing 120. Specifically, when the user uses the applicator 100, by pressing the top wall 1101 on the first housing 110, the automatic implantation of the probe on the analyte sensor 150 and the automatic retraction of the auxiliary needle 161 on the retraction block 160 can be achieved by using the first elastic member 140 and the second elastic member 170.

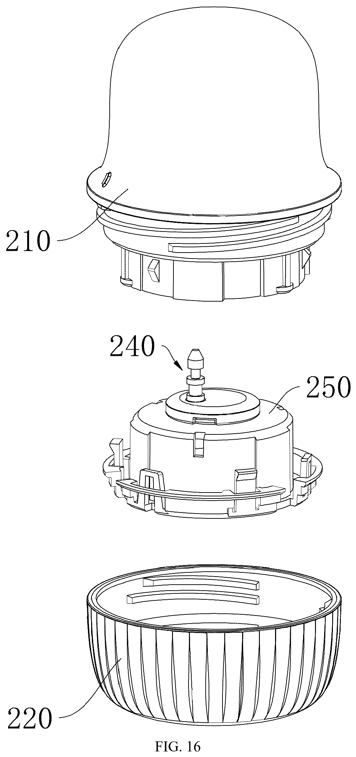

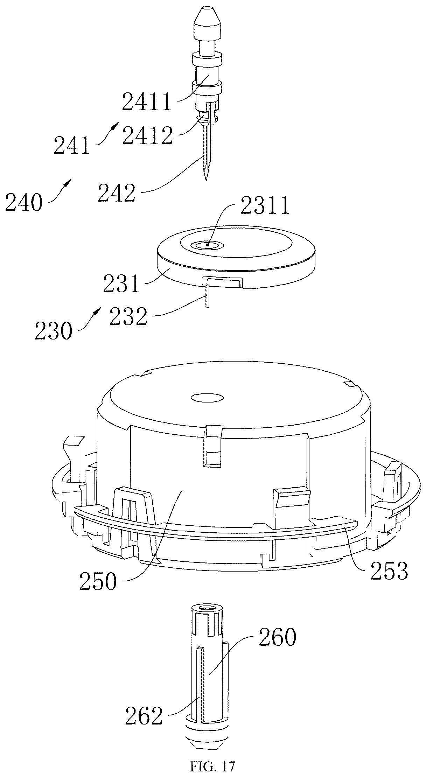

Referring to FIGS. 16 to 18, in a second aspect of the present disclosure, a sterilization unit is provided. The sterilization unit includes an auxiliary needle 240, an analyte sensor 230, and an accommodating member 260. The auxiliary needle 240 includes a handle portion 241 and a sharp portion 242. The handle portion 241 includes a handle section 2411 and a connecting section 2412, and the sharp portion 242 is fixedly arranged on the connecting section 2412. The analyte sensor 230 includes an application portion 231 and a probe 232 that are secured to each other, and the application portion 231 is provided with a through hole 2311. The handle section 2411 forms a seal with the through hole 2311 on a side close to the handle section 2411, and the connecting section 2412 at least partially extends through the through hole 2311, so that the probe 232 is nested in the sharp portion 242. Optionally, the connecting section 2412 extends through the through hole 2311 to the other side of the application portion 231.

The accommodating member 260 is located on the other side of the analyte sensor 230 (i.e., the side of the analyte sensor 230 away from the handle section 2411), and the accommodating member 260 is provided with an accommodating space 261 capable of accommodating the sharp portion 242 and the probe 232. The accommodating member 260 is removably connected to the connecting section 2412 to clamp and fix the application portion 231 between the handle section 2411 and the accommodating member 260. The accommodating member 260 forms a seal with the through hole 2311 on the side away from the handle section 2411 to hermetically isolate the through hole 2311 and the accommodating space 261 from the outside, forming a bacteria barrier.

In this embodiment, the analyte sensor 230 is clamped and fixed through the removable fixation between the auxiliary needle 240 and the accommodating member 260, and the seal is achieved by clamping, thereby hermetically isolating the through hole 2311 and the accommodating space 261 from the outside to form a bacteria barrier. As a result, the fixation and sealing can be independently achieved only by the auxiliary needle 240, the analyte sensor 230, and the accommodating member 260, forming a sterilization unit with a relatively small volume. This enables more sterilization units to be accommodated in the sterilization container during a single sterilization, thereby achieving the effect of improving sterilization efficiency.

The removable fixation herein means that the fixation can be released under certain conditions. For example, the engagement or interference fit can be released when the external force exceeds a certain threshold. As for other special connection structures, for example, locking by thread connection, the locking can be release by rotation when the locking needs to be released.

A seal is formed between the handle section 2411 and the through hole 2311, and between the accommodating member 260 and the through hole 2311. The seal may be formed by two components abutting against each other, or may be formed by matching sealing components.

Specifically, the handle portion 241 and the sharp portion 242 are secured by, for example, insert molding, bonding, or other methods.

In some embodiments, a sealing structure, such as a sealing ring, is further provided between the handle section 2411 and the application portion 231, and between the accommodating member 260 and the application portion 231 to increase the sealing effect of the formed bacteria barrier. In other embodiments, a labyrinth seal or other commonly used sealing methods can also be provided as long as the sealing effect can be increased.

Referring to FIGS. 16 to 18, the sterilization unit further includes a base member 250 located on the other side of the application portion 231. The accommodating member 260 is inserted through the base member 250, and the base member 250 can at least partially shield electron beam radiation, or the base member 250 itself can transmit electron beams, but can cooperate with other parts to shield electron beam radiation.

It can be understood that although electron beam radiation can achieve sterilization without damaging biological enzymes, it may cause irreversible damage to the electronic components inside the application portion 231. Therefore, in order to avoid the failure of the analyte sensor 230 at present, it is usually necessary to separate the application portion 231 from the probe 232 for sterilization, or use a collimator to prevent electron beams from passing through areas other than the sealed space. The former is cumbersome to operate, and the user needs to install it by himself when using it, which affects the user's wearing experience. The latter requires additional collimators and corresponding fixing structures, increasing equipment costs.

In some embodiments, by arranging a base member 250 capable of shielding electron beam radiation on the other side of the application portion 231, and making the accommodating member 260 at least partially extend through the base member 250, the base member 250 can protect the application portion 231 from electron beam radiation damage, and at the same time, can ensure that the probe 232 inside the accommodating member 260 is fully irradiated and sterilized.

In some embodiments, the accommodating member 260 is secured to the base member 250, and the accommodating member 260 can be integrally formed with the base member 250 or fixed by a conventional fixing method such as screwing, engaging or the like.

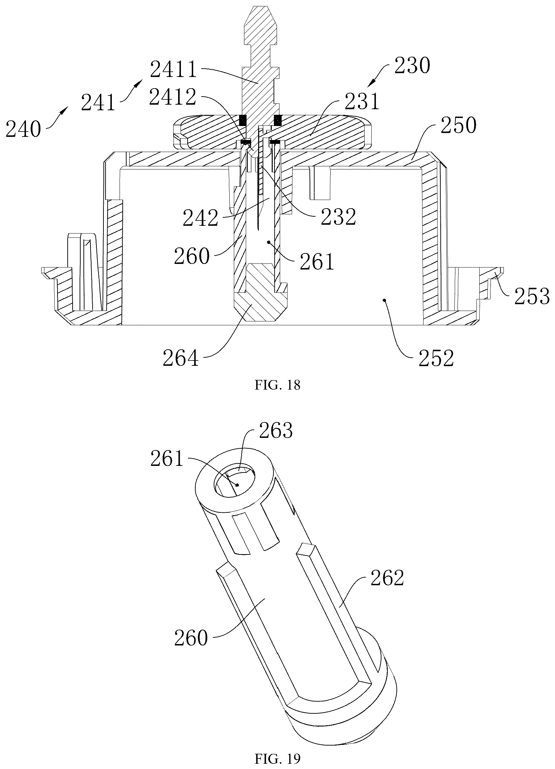

Referring to FIGS. 19 to 20, the outer peripheral surface of the accommodating member 260 is provided with a first guide member 262, which is a guide rib in this embodiment. When the accommodating member 260 is secured to the connecting section 2412, the first guide member 262 abuts against the base member 250 to clamp and secure the base member 250 and the application portion 231 between the handle section 2411 and the accommodating member 260. The separate arrangement of the accommodating member 260 and the base member 250 can reduce the difficulty of injection molding of parts, reduce the occurrence of structural defects of parts, and improve part precision.

Those skilled in the art can understand that the limit of the base member 250 can also be achieved in other ways, for example, when the accommodating member 260 is secured to the connecting section 2412, the sealing portion 264 of the accommodating member 260 is inserted into the base member 250 to limit the base member 250.

Referring to FIGS. 19 to 21, a side of the base member 250 away from the analyte sensor 230 is provided with a second guide member 251 cooperating with the first guide member 262, which is a guide sliding groove in this embodiment. The first guide member 262 and the second guide member 251 can be slidably connected in the axial direction of the accommodating member 260. Through the sliding fit between the first guide member 262 and the second guide member 251, a certain positioning and guiding effect can be achieved for the insertion and installation between the accommodating member 260 and the base member 250, facilitating installation, and also limiting the rotation of the accommodating member 260 relative to the base member 250 in its own circumferential direction, increasing the reliability of clamping and fixing the base member 250 after the accommodating member 260 is engaged with the auxiliary needle 240, and preventing the base member 250 from loosening. Those skilled in the art can understand that the first guide member 262 can also be arranged on the base member 250, and the second guide member 251 can be arranged on the accommodating member 260, i.e., the base member 250 is provided with guide ribs, and the accommodating member 260 is provided with guide sliding grooves, which can also achieve the same function. In addition to guide ribs and guide sliding grooves, any other suitable structures can be used to achieve the sliding fit and abutment between the first guide member and the second guide member.

In some embodiments, a plurality of first guide members 262 are uniformly distributed on the outer peripheral surface of the accommodating member 260 in the circumferential direction to increase guiding stability. Preferably, three first guide members 262 are uniformly distributed on the outer peripheral surface of the accommodating member 260 in the circumferential direction.

Referring to FIGS. 18 to 20, an inner wall of the accommodating space 261 is provided with a first engagement portion 263 at an end close to the analyte sensor 230. The connecting section 2412 can be inserted into the accommodating space 261 and engaged with the first engagement portion 263. The engagement surface after the connecting section 2412 and the first engagement portion 263 are engaged with each other is an inclined surface, so that the engagement and fixation between the two can be released by applying an external force later.

In some embodiments, a plurality of first engagement portions 263 are uniformly distributed on the inner wall of the accommodating space 261 in the circumferential direction to increase engagement stability. Preferably, three first engagement portions 263 are uniformly distributed on the inner wall of the accommodating space 261 in the circumferential direction.

In other embodiments, an annular groove is provided at an end of the connecting section 2412 close to the sharp portion 242, and an end of the accommodating member 260 close to the analyte sensor 230 is adapted to be inserted into the annular groove of the connecting section 2412 to connect the accommodating member 260 to the connecting section 2412. An outer wall of the insertion end of the accommodating member 260 inserted into the connecting section 2412 is provided with an engagement portion, and the annular groove is provided with an engagement surface adapted to be engaged and fixed with the engagement portion. Optionally, the engagement portion can be provided in the annular groove, and the engagement surface can be provided on the outer wall of the insertion end of the accommodating member 260 inserted into the connecting section 2412.

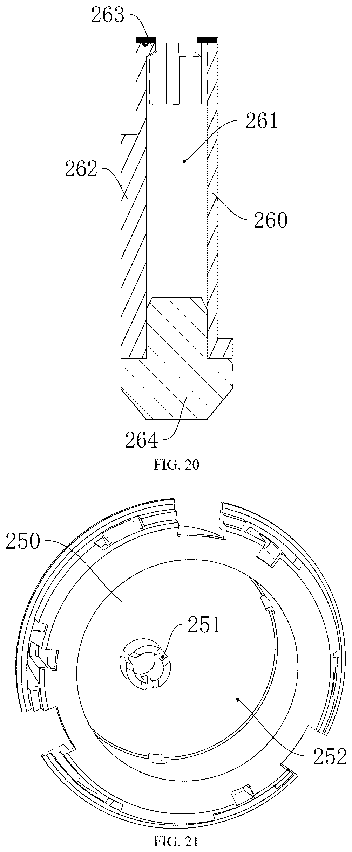

Referring to FIGS. 18 and 20, the accommodating space 261 extends through the accommodating member 260, and the accommodating member 260 further includes a sealing portion 264 capable of being secured to the accommodating member 260 and closing an opening of the accommodating space 261 at an end away from the analyte sensor 230. The separate arrangement of the sealing portion 264 and the accommodating member 260 can also reduce the difficulty of injection molding of parts, reduce the occurrence of structural defects of parts, and improve part precision.

The fixing method between the sealing portion 264 and the accommodating member 260 includes but is not limited to conventional fixing methods such as interference fit, bonding, welding, and screw connection. In other embodiments, the sealing portion 264 can also be integrally formed with the accommodating member 260, such as injection molding.

In some embodiments, the sealing portion 264 is made of a material that can transmit radiation electron beams, and other parts of the accommodating member 260 and the base member 250 can all shield electron beams, so as to further increase the directionality of the electron beams entering the accommodating space 261 and further reduce the possibility of electron beam radiation damage.

Referring to FIG. 18, the base member 250 can transmit radiation electron beams, and a shielding cavity 252 capable of mounting a shielding block is provided on a side of the base member 250 away from the analyte sensor 230. After mounting, the accommodating member 260 can receive electron beam irradiation to ensure that the probe 232 in the accommodating member 260 can be fully irradiated and sterilized.

In the actual sterilization process, a shielding block is mounted into the shielding cavity 252 before sterilization, and the shielding block is taken out after sterilization. The shielding block can be reused in the sterilization step, thereby effectively reducing the cost of the sterilization unit. Specifically, the shielding block itself has a channel through which electron beams can pass. The projection of the channel in the axial direction of the accommodating member 260 at least covers the accommodating member itself. After the shielding block is mounted, the accommodating member 260 is at least partially located in the channel.

In some embodiments, at least a part of the base member 250 has a shielding layer, which can cooperate with the shielding block or individually shield electron beams. The superimposed projection of the shielding layer and the shielding block in the axial direction of the accommodating member 260 at least completely covers the analyte sensor 230. The shielding layer can be a lead layer or other material layer capable of shielding electron beam radiation. When the base member 250 individually shields electron beams, the projection of the shielding layer in the axial direction of the accommodating member 260 completely covers the analyte sensor 230. When at least a part of the base member 250 has a shielding layer and cooperates with the shielding block, the superimposed projection of the shielding layer and the shielding block in the axial direction of the accommodating member 260 completely covers the analyte sensor 230. The projection of the shielding layer in the axial direction of the accommodating member 260 and the projection of the shielding member in the axial direction of the accommodating member 260 can form a complementary structure, or at least partially overlap. The combined projection of the two only needs to completely cover the analyte sensor 230.

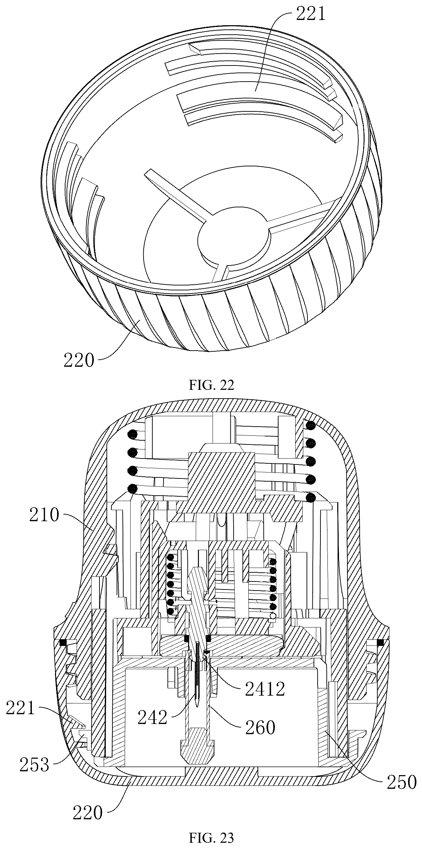

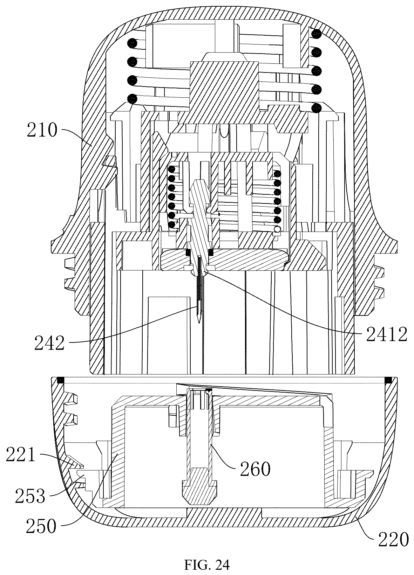

Referring to FIGS. 16, 22 to 24, in a third aspect of the present disclosure, an applicator is provided. The applicator includes a housing 210, a bottom cover 220, and the sterilization unit according to the above second aspect. The housing 210 is removably connected to the bottom cover 220 to accommodate the sterilization unit inside the housing 210 and the bottom cover 220. The handle section 2411 and the analyte sensor 230 are respectively removably secured to the housing 210, and the accommodating member 260 is relatively fixed to the bottom cover 220 to limit the movement of the accommodating member 260 toward the side close to the housing 210 relative to the bottom cover 220.

Through the fixation between the housing 210 and the bottom cover 220, a second layer of sealing can be achieved, further increasing the sealing effect in the accommodating space 261. The handle section 2411 and the analyte sensor 230 are respectively removably secured to the housing 210, which can prevent the analyte sensor 230 from falling off when the housing 210 is removed from and the bottom cover 220.

For ease of description, the tightness mentioned below refers to the threshold force required to release the fixation of the removable fixing structure.

The tightness of the removable fixation between the handle section 2411 and the analyte sensor 230 and the housing 210 is greater than the tightness of the removable fixation between the accommodating member 260 and the connecting section 2412, so that when the housing 210 is removed from the bottom cover 220, the accommodating member 260 is preferentially disengaged from the connecting section 2412, thereby pulling the sharp portion 242 and the probe 232 out of the accommodating member 260.

In some embodiments, the housing 210 is connected to the bottom cover 220 by threads. The threads in the bottom cover 220 are divided into multiple parts in the circumferential direction to reduce the manufacturing difficulty of the bottom cover 220 while ensuring the thread connection and fixation with the housing 210.

In some embodiments, a sealing ring is also provided between the housing 210 and the bottom cover 220 to increase the sealing effect. On the one hand, it can prevent dust or water from entering the housing 210, and on the other hand, it can further increase the sealing performance inside the accommodating member 260. Those skilled in the art can understand that a labyrinth seal or other commonly used sealing methods can also be provided as long as the sealing effect can be increased.

Referring to FIGS. 16 and 22 to 24, the sterilization unit further includes a base member 250 located on the other side of the application portion 231. The base member 250 is capable of limiting the movement of the accommodating member 260 toward the side close to the analyte sensor 230 along its own axial direction. An inner wall of the bottom cover 220 is provided with a second engagement portion 221, and an outer periphery of the base member 250 is provided with a third engagement portion 253. When the housing 210 is secured to the bottom cover 220, the second engagement portion 221 is engaged with the third engagement portion 253.

The tightness of the engagement between the second engagement portion 221 and the third engagement portion 253 is also greater than the tightness of the removable fixation between the accommodating member 260 and the connecting section 2412 to ensure that the sharp portion 242 and the probe 232 can be pulled out of the accommodating member 260.

In this embodiment, an accommodating space 261 for accommodating the probe 232 of the analyte sensor 230 and hermetically isolating it from the outside is formed between the auxiliary needle 240, the analyte sensor 230, and the accommodating member 260.

For conventional analyte sensors, the accommodating member is usually part of the bottom cover or directly in physical contact with the bottom cover. The bottom cover directly drives the accommodating member to be separated from the auxiliary needle and/or the analyte sensor, resulting in the need to carry out sterilization together with the bottom cover during irradiation sterilization. The bottom cover occupies a lot of space, leading to a small number of analyte sensors that can be accommodated in each irradiation sterilization, low sterilization efficiency, and the structure of the bottom cover is relatively complex.

In some embodiments, by arranging the accommodating member 260 separately from the bottom cover 220, there is no need for direct contact or matching physical structures between the two, so that the bottom cover 220 has no direct coupling relationship with the accommodating member 260, which effectively reduces the structural complexity of the bottom cover 220. During irradiation sterilization, there is no need to carry out sterilization together with the bottom cover 220, which effectively improves the sterilization efficiency.

In some embodiments, the accommodating space 261 can be formed by the pressing force of other parts on the auxiliary needle 240 and the accommodating member 260. In other embodiments, the accommodating space 261 can also be independently formed by the connection relationship between the auxiliary needle 240, the analyte sensor 230, and the accommodating member 260.

It can be understood that the sterilization unit described in the second aspect of the present disclosure can be applied to the applicator described in the first aspect of the present disclosure. Specifically, the analyte sensor 150 and the housing cover 180 described in the first aspect can be replaced with the analyte sensor 230 and the bottom cover 220 described in the second aspect, and the auxiliary needle 240, the base member 250, the accommodating member 260, and other components described in the second aspect can be added accordingly.

The technical features of the above-described embodiments can be arbitrarily combined. To make the description concise, all possible combinations of the technical features in the above-described embodiments are not described. However, as long as there is no contradiction between the combinations of these technical features, they should be considered as being within the scope of the description.

Although the present disclosure is disclosed above with preferred embodiments, the above embodiments are not intended to limit the present disclosure. For any person skilled in the art, without departing from the scope of the technical solution of the present disclosure, many possible changes and modifications can be made to the technical solution of the present disclosure by using the technical content disclosed above, or modified into equivalent embodiments with equivalent changes. Therefore, any simple modifications, equivalent changes, and modifications made to the above embodiments according to the technical essence of the present disclosure without departing from the content of the technical solution of the present disclosure shall still fall within the scope of the technical solution of the present disclosure.

## Claims

1. An applicator (100), comprising:
a first housing (110) comprising a top wall (1101);
a second housing (120) partially accommodated in the first housing (110), wherein a trigger member (111) is provided on a side of the top wall (1101) of the first housing (110) facing the second housing (120), and a limiting member (121) is provided on the second housing (120); and
a pushing block (130) slidably connected to the second housing (120), wherein a trigger mating member (131) is provided on the pushing block (130);
wherein the trigger mating member (131) is capable of abutting against both the trigger member (111) and the limiting member (121), and the trigger member (111) is capable of driving the trigger mating member (131) to be disengaged from the limiting member (121) when driven by the first housing (110).

2. The applicator (100) according to claim 1, wherein the trigger member (111) is in positional interference with the trigger mating member (131); and
wherein the trigger mating member (131) has a first state where the trigger mating member (131) is limited by the limiting member (121), and a second state where the trigger mating member (131) is capable of being disengaged from the limiting member (121) when pushed by the trigger member (111).

3. The applicator (100) according to claim 2, wherein one of the trigger member (111) and the trigger mating member (131) is configured with a hook (1111), and the other of the trigger member (111) and the trigger mating member (131) is configured with a groove (1311), and the hook (1111) is capable of being engaged with the groove (1311).

4. The applicator (100) according to any one of claims 1 to 3, wherein a fastening member (112) is further provided on the top wall (1101), a fastening mating member (122) is provided on the second housing (120), the fastening member (112) abuts against the fastening mating member (122) in a movement direction of the trigger member (111), and the fastening member (112) is capable of driving the fastening member (112) and/or the fastening mating member (122) to elastically deform when driven by the top wall (1101).

5. The applicator (100) according to any one of claims 1 to 3, wherein a first positioning member (113) is further provided in the first housing (110), a first positioning mating member (123) is provided on the second housing (120), and the first positioning member (113) is in sliding fit with the first positioning mating member (123) in a movement direction of the trigger member (111).

6. The applicator (100) according to any one of claims 1 to 3, wherein a second positioning member (132) is further provided on the pushing block (130), a second positioning mating member (124) is provided on the second housing (120), and the second positioning member (132) is in sliding fit with the second positioning mating member (124) in a movement direction of the trigger member (111).

7. The applicator (100) according to any one of claims 1 to 3, further comprising:
a retraction block (160) movably mounted on the pushing block (130); and
an analyte sensor (150) mounted on the pushing block (130) and comprising a probe (151),
wherein the retraction block (160) is provided with an auxiliary needle (161), the auxiliary needle (161) is formed with an accommodating cavity (1611), and the probe (151) is at least partially accommodated in the accommodating cavity (1611).

8. The applicator (100) according to claim 7, wherein an elastic claw (134) is further provided on the pushing block (130), and the elastic claw (134) is capable of abutting against an inner peripheral wall (1201) of the second housing (120); and
wherein the elastic claw (134) is in positional interference with the retraction block (160), and the elastic claw (134) is configured to elastically deform in a direction away from the retraction block (160) in response to pushing by the retraction block (160).

9. The applicator (100) according to claim 8, wherein the elastic claw (134) comprises a pushing inclined surface (1341), and the pushing inclined surface (1341) abuts against the retraction block (160) in a movement direction of the retraction block (160) relative to the pushing block (130); and
a sliding groove (1621) is provided on an outer peripheral wall (162) of the retraction block (160), and the elastic claw (134) is in sliding fit with the sliding groove (1621) in the movement direction of the retraction block (160) relative to the pushing block (130).

10. A sterilization unit, comprising:
an auxiliary needle (240) comprising a handle portion (241) and a sharp portion (242), wherein the handle portion (241) comprises a handle section (2411) and a connecting section (2412), and a tip of the sharp portion (242) is away from the connecting section (2412);
an analyte sensor (230) comprising an application portion (231) and a probe (232) secured to each other, wherein the application portion (231) is provided with a through hole (2311), the handle section (2411) forms a seal with the through hole (2311) on a side close to the handle section (2411), and the connecting section (2412) at least partially extends through the through hole (2311); and
an accommodating member (260) located on a side of the analyte sensor (230) away from the handle section (2411), wherein the accommodating member (260) is provided with an accommodating space (261) capable of accommodating the sharp portion (242) and the probe (232);
wherein the accommodating member (260) is removably secured to the connecting section (2412), and the accommodating member (260) forms a seal with the through hole (2311) on a side away from the handle section (2411).

11. The sterilization unit according to claim 10, further comprising a base member (250) located on a side of the application portion (231) away from the handle portion (241), wherein the accommodating member (260) is at least partially inserted through the base member (250).

12. The sterilization unit according to claim 11, wherein the accommodating member (260) is integrally formed with the base member (250) or secured to the base member (250).

13. The sterilization unit according to claim 12, wherein one of the accommodating member (260) and the base member (250) is provided with a first guide member (262), and the other of the accommodating member (260) and the base member (250) is provided with a second guide member (251) cooperating with the first guide member (262); and wherein when the accommodating member (260) is secured to the connecting section (2412), the first guide member (262) abuts against the second guide member (251).

14. The sterilization unit according to any one of claims 10 to 13, wherein an inner wall of the accommodating space (261) is provided with a first engagement portion (263) at an end close to the analyte sensor (230); and when the accommodating member (260) is secured to the connecting section (2412), the connecting section (2412) is engaged with the first engagement portion (263).

15. The sterilization unit according to claim 14, wherein the accommodating space (261) extends through the accommodating member (260), and the accommodating member (260) further comprises a sealing portion (264) capable of being secured to the accommodating member (260) to seal an opening of the accommodating space (261) at an end away from the analyte sensor (230).

16. The sterilization unit according to any one of claims 11 to 13, wherein a shielding cavity (252) capable of mounting a shielding block is provided on a side of the base member (250) away from the analyte sensor (230).

17. The sterilization unit according to any one of claims 11 to 13, wherein the base member (250) comprises a shielding layer, and a projection of the shielding layer in an axial direction of the accommodating member (260) at least partially covers the analyte sensor (230).

18. An applicator, comprising:
a housing (210);
a bottom cover (220) removably secured to the housing (210); and
the sterilization unit according to any one of claims 10 to 17, accommodated inside the housing (210) and the bottom cover (220);
wherein the handle section (2411) and the analyte sensor (230) are respectively removably secured to the housing (210), and the accommodating member (260) is relatively fixed to the bottom cover (220).

19. The applicator according to claim 18, wherein when the sterilization unit comprises a base member (250), the base member (250) is capable of limiting a movement of the accommodating member (260) toward a side close to the analyte sensor (230) in an axial direction of the accommodating member (260); and
an inner wall of the bottom cover (220) is provided with a second engagement portion (221), and an outer periphery of the base member (250) is provided with a third engagement portion (253); and when the housing (210) is secured to the bottom cover (220), the second engagement portion (221) is engaged with the third engagement portion (253).

20. An applicator, comprising:
a first housing (110) comprising a top wall (1101);
a second housing (120) partially accommodated in the first housing (110), wherein a trigger member (111) is provided on a side of the top wall (1101) of the first housing (110) facing the second housing (120), and a limiting member (121) is provided on the second housing (120);
a pushing block (130) slidably connected to the second housing (120), wherein a trigger mating member (131) is provided on the pushing block (130); and
the sterilization unit according to any one of claims 10 to 17, mounted on the pushing block (130);
wherein the trigger mating member (131) is capable of abutting against both the trigger member (111) and the limiting member (121), and the trigger member (111) is capable of driving the trigger mating member (131) to be disengaged from the limiting member (121) when driven by the first housing (110).

21. An applicator with a sterilization unit, comprising the applicator (100) according to any one of claims 1 to 9, and the sterilization unit according to any one of claims 10 to 17.
